# EUROPEAN PATENT APPLICATION

(11) **EP 2 754 435 A1**
(43) Date of publication of application: **16.07.2014**
(21) Application number: 13177243.6
(22) Date of filing: 19.07.2013
(51) Int. Cl.: A61K 9/00, A61K 39/12, A61K 47/48

(54) **Biodegradable carrier with adjustable zeta potentials and particle sizes, method for making the same, and pharmaceutical composition comprising the same**

(30) Priority: 09.01.2013 TW 102100788
(71) Applicant: National Cheng Kung University, Tainan City (TW)
(72) Inventor: Lin, Yee-Shin, TAINAN CITY (TW); Chen, Yu-Hung, TAINAN CITY (TW)
(74) Representative: Lang, Christian

(57) **Abstract**

The present invention is related to a biodegradable carrier with adjustable zeta potentials and particle sizes, a method for making the same, and a pharmaceutical composition comprising the same. In such a method, a first solution comprising a first biodegradable macromolecule is prepared, and a second solution comprising a second biodegradable macromolecule is also prepared according to a desired zeta potential of a biodegradable carrier and further added into the first solution to form a mixture solution. The biodegradable carrier with the desired zeta potentials is formed by the attraction force between the different electric properties. Then, the mole number of the first biodegradable macromolecule and the second biodegradable macromolecule in the mixture solution are proportionally adjusted according to a desired particle size of the biodegradable carrier. Therefore, the zeta potential and the particle size of the biodegradable carrier are adjustable artificially.

SEQUENCE LISTING

<110>  National Cheng Kung University

<120>  Dengue Vaccine, Medicinal Composition Comprising the Same,
       Nucleotide Sequence, and Antibody Composition

<130>  N/A

<160>  1

<170>  PatentIn version 3.5

<210>  1

<211>  352

<212>  PRT

<213>  Dengue Virus & Japanese Encephalitis Virus

<400>  1

Asp Ser Gly Cys Val Val Ser Trp Lys Asn Lys Glu Leu Lys Cys Gly
1               5                   10                  15

Ser Gly Ile Phe Ile Thr Asp Asn Val His Thr Trp Thr Glu Gln Tyr
            20                  25                  30

Lys Phe Gln Pro Glu Ser Pro Ser Lys Leu Ala Ser Ala Ile Gln Lys
        35                  40                  45

Ala His Glu Glu Gly Ile Cys Gly Ile Arg Ser Val Thr Arg Leu Glu
    50                  55                  60

Asn Leu Met Trp Lys Gln Ile Thr Pro Glu Leu Asn His Ile Leu Ser
65                  70                  75                  80

Glu Asn Glu Val Lys Leu Thr Ile Met Thr Gly Asp Ile Lys Gly Ile
                85                  90                  95

Met Gln Ala Gly Lys Arg Ser Leu Arg Pro Gln Pro Thr Glu Leu Lys
            100                 105                 110

Tyr Ser Trp Lys Thr Trp Gly Lys Ala Lys Met Leu Pro Thr Glu Ser
        115                 120                 125

His Asn Gln Thr Phe Leu Ile Asp Gly Pro Glu Thr Ala Glu Cys Pro
    130                 135                 140

Asn Thr Asn Arg Ala Trp Asn Ser Leu Glu Val Glu Asp Tyr Gly Phe
145                 150                 155                 160

Gly Val Phe Thr Thr Asn Ile Trp Leu Lys Leu Arg Glu Lys Gln Asp
                165                 170                 175

Val Phe Cys Asp Ser Lys Leu Met Ser Ala Ala Ile Lys Asp Asn Arg
            180                 185                 190

Ala Val His Ala Asp Met Gly Tyr Trp Ile Glu Ser Ala Leu Asn Asp
        195                 200                 205

Thr Trp Lys Ile Glu Lys Ala Ser Phe Ile Glu Val Lys Ser Cys His
    210                 215                 220

Trp Pro Lys Pro His Thr Leu Trp Ser Asn Gly Val Leu Glu Ser Glu
225                 230                 235                 240

Met Ile Ile Pro Lys Asn Phe Ala Gly Pro Val Ser Gln His Asn Tyr
                245                 250                 255

Arg Pro Gly Tyr His Thr Gln Thr Ala Gly Pro Trp His Leu Asn Gly
            260                 265                 270

Ile Val Leu Asp Phe Asp Tyr Cys Pro Gly Thr Lys Val Thr Ile Thr
        275                 280                 285

Glu Asp Cys Gly Lys Arg Gly Pro Ser Val Arg Thr Thr Thr Asp Ser
    290                 295                 300

Gly Lys Leu Ile Thr Asp Trp Cys Cys Arg Ser Cys Ser Leu Pro Pro
305                 310                 315                 320

Leu Arg Phe Arg Thr Glu Asn Gly Cys Trp Tyr Gly Met Glu Ile Arg
                325                 330                 335

Pro Val Arg His Asp Glu Thr Thr Leu Val Arg Ser Gln Val Asp Ala
            340                 345                 350

## Description

### BACKGROUND OF THE INVENTION

### 1. Field of the Invention

The present invention relates a biodegradable carrier with adjustable zeta potentials and particle sizes, a method for making the same, and a pharmaceutical composition comprising the same. More particularly, a method for making a carrier with adjustable adjusting the zeta potential and the particle size not only complies with the requirement of the application, but also makes the zeta potentials and the particle sizes of the carrier uniform, so that the pharmaceutical composition comprising the carrier may significantly produces its effect through penetration or phagocytosis by macrophage easily.

### 2. Description of Related Art

Many researches reported that biomacromolecules can be used as drug carriers for delivering drugs. In general, a drug carrier can hold molecular drug or protein to improve many diseases which can not be cured by the traditional medicine. For example, liposome is used as a drug carrier in drug delivery system for many compounds, such as pharmaceutical active compound, diagnostic substance, and cosmetics. Up to now, the liposome is one of most potential drug carriers. The liposome is a tiny bubble with one-bilayer or multi-bilayer structure, which encapsulates a hydrophilic region inside a hydrophobic membrane so that a dissolved hydrophilic substance can be held in the liposome, and a hydrophobic substance can be dissolved into the membrane. The bilayer composition of the liposome is similar as that of organism's cell membrane and has biocompatibility and biodegradability, so the liposome is widely used as a drug carrier in drug delivery application.

Adjuvant is applied in vaccine over 60 years. The adjuvant action mechanism generally comprises: (a) increasing the life or the immunity of an antigen in the vaccine, (b) delivering antigen to the antigen-presenting cell, (c) improving antigen display in antigen-presenting cell, and (d) inducing the production of immunoregulatory cytokine. The adjuvant type is divided into inorganic salt, delivery system, bacterial derivative, and natural mediator. Besides the aluminum salts (aluminum hydroxide and aluminum phosphate) actually can be used on the human, the liposome is mostly applied to the several human subject trial and cancer cell line test because the liposome can be fused into almost any organism structures. The liposome is applied to many human subject trials on many cancer diseases, acquired immune deficiency syndrome (AIDS), viral and bacterial infectious diseases, multiple myeloma, kaposi sarcoma, cryptococcosis meningitis and anti-fungal effect, and is applied to be as a carrier for erythropoietin and drugs delivery.

The foregoing applications and developments of the liposome have great value in biology or pharmaceutical medicine. However, the zeta potential and the particle size of the liposome produced by traditional methods for preparation of lipsome can not be controlled accurately that results in the large diversity of the zeta potential and the particle size. Therefore, the liposome is aggregated easily because of the attraction between the liposome molecules and is fused together easily, which will further results in a fusion phenomenon. After the aggregation of the liposome, the contact site between two liposomes is unstable and defected to promote liposomes to be fused together as a large particle. In most cases, the fusion phenomenon incurs at the condition that a large liposome fuses with a small liposome, so the particle size of the liposome gets larger. The larger liposome is absorbed by cell uneasily so that the utility of the liposome in drug delivery is decreased and the development of liposome application is influenced.

Up to now, the liposome can be produced by methods as thin-film hydration, organic solvent injection, or detergent dialysis. However, no matter what method described above has a solvent evaporation step and a hydration step which result in greatly increasing the production cost. In addition, the liposome has a single-bilayer or multi-bilayer structure so that the liposome needs to be kept in a suspension and is uneasily kept in a dry powder form, which results in the increase of the shipping cost.

### SUMMARY OF THE INVENTION

In view of the foregoing disadvantages of the traditional macromolecular (drug, protein etc.,) carrier in actual implementation, the object of the present invention is to provide a method for making a biodegradable carrier with adjustable zeta potential and particle size. The method comprises the following steps. A first solution comprising a first biodegradable macromolecule with a first electric property is prepared. Next, according to a desired zeta potential of a biodegradable carrier, a second solution comprising a second biodegradable macromolecule with a second electric property is prepared and added into the first solution to form a mixture solution. The first electric property is opposite to the second electric property for forming the biodegradable carrier with the desired zeta potential by attraction force between the different electric properties. Then, according to a desired particle size of the biodegradable carrier, the mole number of the first biodegradable macromolecule and the second biodegradable macromolecule in the mixture solution are proportional adjusted. The particle size of the biodegradable carrier is positively correlated with the mole number of a solute in the mixture solution for making the biodegradable carrier with desired particle size. Finally, the mixture solution is filtrated to obtain the biodegradable carrier. Therefore, the zeta potential and the particle size of the biodegradable carrier are adjustable through setting the zeta potential of the biodegradable carrier first and then adjusting the concentration of the mixture solution for forming the biodegradable carrier with desired particle size by the foregoing making method in order that there are different strategies for meeting the need of different carried substances with different physical and chemical properties.

Furthermore, the zeta potential and the particle size of the biodegradable carrier obtained by the making method of the present invention is more uniform, so the biodegradable carrier has better dispersibility in the mixture solution and is not aggregated during a dry process. Therefore, the biodegradable carrier can be kept in the form of dry powder for resolving the problem that the liposome needs to be kept in a suspension form and decreasing the shipping cost.

According to an embodiment of the present invention, the first biodegradable macromolecule is heparin or polyglutamic acid with negative charge. The second biodegradable macromolecule is chitosan or collagen with positive charge.

According to another embodiment of the present invention, a carried substance is added into the first solution or the second solution for making the carried substance be held in the biodegradable carrier. The foregoing carried substance is selected from the group consisting of nucleic acid, short peptide, protein drugs, small molecular compounds, viruses, bacterium, and cells. The electric property of the carried substance is the same as that of the solution which the carried substance is added into.

Another object of the present invention is to provide a biodegradable carrier with adjustable zeta potential and particle size. The particle size of the biodegradable carrier is between micrometers to nanometers. According to another embodiment, the particle size of the biodegradable carrier is between 10 µm to 40 nm, and the zeta potential of the biodegradable carrier is between +35mv to -35mv. Moreover, the biodegradable carrier is used for holding a carried substance.

A pharmaceutical composition is also provided. The pharmaceutical composition comprises a foregoing biodegradable carrier with adjustable zeta potential and particle size, a carried substance, and at least one pharmaceutically acceptable vehicle. The carried substance is held in the biodegradable carrier and is selected from the group consisting of nucleic acid, short peptide, protein drugs, small molecular compounds, viruses, bacterium, and cells. Because the zeta potential and the particle size of the biodegradable carrier is adjustable, not only can the release time of the carried substance be controlled, but also the pharmaceutical composition comprising the biodegradable carrier can stimulate specific immune responses to enhance the immune responses to the specific antigen for increasing the protection efficiency of the vaccine. Moreover, the zeta potential and the particle size of the biodegradable carrier obtained by the method of the present invention are uniform, so the aggregation and fusion of the traditional liposome due to the large diversity of the particle size and the zeta potential of the traditional liposome is prevented. Accordingly, the pharmaceutical composition comprising the biodegradable carrier of the present invention produces the effect of the carried substance through penetration or phagocytosis by macrophage easily and releases the carried substance due to the biodegradability of the biodegradable carrier.

According to the other embodiment of the present invention, the pharmaceutical composition is used for producing a vaccine or a drug for treating or preventing hemorrhagic dengue fever or dengue shock syndrome.

### BRIEF DESCRIPTION OF THE DRAWINGS

Fig. 1 is a flow chart of a method for making a biodegradable carrier with adjustable zeta potential and particle size according to one embodiment of the present invention;
Fig. 2 shows the analysis results of the mean size of the biodegradable carrier under different concentrations (1X-10X) of the mixture solution containing chitosan and polyglutamic acid in another embodiment of the present invention; and
Fig. 3 shows the analysis results of the mean size of the biodegradable carrier under different concentrations (20X-35X) of the mixture solution containing chitosan and polyglutamic acid in the other embodiment of the present invention.

### DETAILED DESCRIPTION OF THE PREFERRED EMBODIMENT

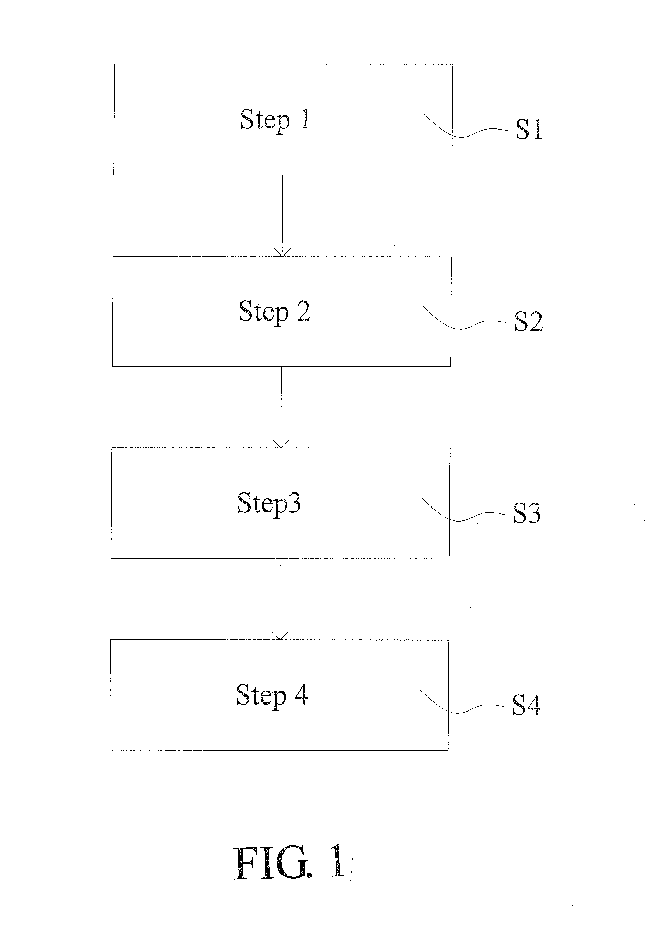

Fig.1 is a flow chart of a method for making a biodegradable carrier with adjustable zeta potentials and particle sizes according to one embodiment of the present invention. The method comprises the following steps:

Step 1 (S1): a first solution comprising a first biodegradable macromolecule with a first electric property is prepared. The first biodegradable macromolecule is heparin (HP) or polyglutamic acid (γ-PGA) with negative charge.

Step 2 (S2): according to a desired zeta potential of a biodegradable carrier, a second solution comprising a second biodegradable macromolecule with a second electric property is prepared and added into the first solution to form a mixture solution. The first electric property is opposite to the second electric property for forming the biodegradable carrier with the desired zeta potential by attraction force between the different electric properties. The second biodegradable macromolecule is chitosan (CS) or collagen with positive charge.

Step 3 (S3): according to a desired particle size of the biodegradable carrier, the mole number of the first biodegradable macromolecule and the second biodegradable macromolecule in the mixture solution are proportionally adjusted. The particle size of the carrier is positively correlated with the mole number of a solute in the mixture solution for making the biodegradable carrier with a desired particle size.

Step 4 (S4): the mixture solution from Step 3 (S3) is filtrated to obtain the biodegradable carrier with an adjustable zeta potentials and particle sizes. The particle size of the biodegradable carrier is between micrometers to nanometers. According to an embodiment of the present invention, the particle size of the biodegradable carrier is between 10 µm to 40 nm, and the zeta potential of the biodegradable carrier is between +35mv to -35mv.

Moreover, in the foregoing method, a carried substance can be added into the first solution or the second solution before the Step 3, so that the biodegradable carrier obtained by the foregoing method holds the carried substance. The carried substance is selected from the group consisting of nucleic acid, short peptide, protein drugs, small molecular compounds, viruses, bacterium, and cells. The nucleic acid is deoxyribonucleic acid (DNA), ribonucleic acid (RNA), or peptide nucleic acids (PNA). The electric property of the carried substance is the same as that of the solution into which the carried substance is added.

The method of the biodegradable carrier of the present invention could be performed by the following examples to further show the range of the actual application, but not to limit the spirit of the present invention:

### Example 1: Prepare a first solution comprising a first biodegradable macromolecule

In detail, the first biodegradable macromolecule is polyglutamic acid. A proper amount of polyglutamic acid is added into de-ionized water and stirred by electromagnetic stirrer until the polyglutamic acid is totally dissolved. Then, the polyglutamic acid solution is removed the sodium by membrane dialysis. The dialysis process is accomplished at 4 °C for preventing bacterial growth. After dialysis, the polyglutamic acid solution is put at -20°C for being totally frozen. Then, the water content of the frozen polyglutamic acid solution is removed by lyophilization to obtain the crystallized powder of the polyglutamic acid. The crystallized powder of the polyglutamic acid is stored in a sterilized tube and put in a moisture-proof box. Finally, a proper amount of crystallized powder of the polyglutamic acid is taken and dissolved in the de-ionized water in a desired concentration, which is the first solution comprising the first macromolecule with negative charge.

However, one skilled in the art will readily recognize that the foregoing method for preparation of the first solution is one of embodiments. After reading and understanding the descriptions of the present invention, it will be obvious to those skilled in the art that various modifications may be made and not limited to the foregoing embodiment.

### Example 2: Prepare a second solution comprising a second biodegradable macromolecule

According to a desired zeta potential of a biodegradable carrier, a second solution containing a second biodegradable macromolecule is prepared and added into the first solution. In detail, the second biodegradable macromolecule is chitosan. 5g low-viscous chitosan is added into 495 ml de-ionized water with 5 ml glacial acetic acid and stirred by electromagnetic stirrer until the chitosan solution stays in a yellow and pellucid state. It is worth noted that NH₂ of the chitosan is converted to NH₃⁺ under an acidic condition, so the chitosan is positively charged. Moreover, the acetylation degree of the chitosan influences the ratio of the positive electric charge on the chitosan. For example, when the acetylation degree of the chitosan is 100%, the NH₂ of the chitosan is totally converted to NH₃⁺; however, if the acetylation degree of the chitosan is less than 100%, there are acetyl groups on the chitosan, and those acetyl groups are not positively charged under an acidic condition, so the total quantity of positive charge of the chitosan is decreased. Accordingly, more chitosan must be added into the de-ionized water with glacial acetic acid to reach the desired quantity of positive charge in the chitosan solution.

Next, the glacial acetic acid in the chitosan solution is removed by membrane dialysis. The pH of the chitosan solution is about 6.5 after dialysis. Then, the chitosan solution is filtrated by air suction filter to remove the impurity. Finally, the chitosan solution is heated and stirred at 135 °C for being concentrated until the concentration of the chitosan solution reaches 20-30 mg/ml, which is the second solution comprising the second macromolecule with positive charge.

Similarly, one skilled in the art will readily recognize that the foregoing method for preparation of the second solution is one of embodiments. After reading and understanding the descriptions of the present invention, it will be obvious to those skilled in the art that various modifications may be made and not limited to the foregoing embodiment.

It is noted that the foregoing first and second biodegradable macromolecule can be natural macromolecules, such as heparin or polyglutamic acid, and chitosan or collgen, respectively. The foregoing first and second biodegradable macromolecule also can be artificial biodegradable macromolecules.

### Example 3: Form and filtrate a mixture solution to obtain a biodegradable carrier

5 ml polyglutamic acid solution from Example 1 is taken and mixed with 6 ml chitosan solution from Example 2 to form a 11 ml mixture solution, and the mixture solution is stirred for 2 minutes. In the mixture solution, the total dry weight of the polyglutamic acid and the chitosan is 2 mg. The concentration of the foregoing mixture solution is as one-fold (1X) concentration to be the standard concentration of the mixture solution in the following experiment. The 11 ml mixture solution is filtrated to obtain the biodegradable carrier. The zeta potential of the biodegradable carrier is 13 mV, and the particle size of the same is 40 nm.

Table 1 shows the charge ratio and the weight ratio of chitosan (CS) to polyglutamic acid (γ-PGA), and the particle size and the zeta potential of the foregoing biodegradable carrier. The N/A means that the data is undetectable by dynamic light scattering (DLS) because of the precipitation of the biodegradable carrier. The type of DLS is Zetasizer Nano Serie (3000HS, Malvern Instruments, Worcestershire, UK).

**Table 1**

| Charge ratio CS: γ-PGA | Weight ratio CS: γ-PGA | Size (nm) | Zeta potential (mV) |
|---|---|---|---|
| 4:1 | 1.704:0.296 | 40.49±0.8415 | 13.2±0.354 |
| 3:1 | 1.624:0.376 | N/A | N/A |
| 2:1 | 1.484:0.516 | N/A | N/A |

As shown in Table 1, the biodegradable carrier made under the charge ratio of CS to γ-PGA being 4:1 in 1X concentration of mixture solution is stable. The precipitation was happened while the chitosan solution and the polyglumatic acid were mixed under the other charge ratios of CS to γ-PGA, such as 3:1 or 2:1.

### Example 4: Proportionally adjust the mole number of solute in the mixture solution

According to the desired particle size of the biodegradable carrier, the mole number of the first biodegradable macromolecule and the second biodegradable macromolecule is proportionally adjusted in the mixture solution. Based on the method for preparing 1X mixture solution, five-fold (5X) and ten-fold (10X) mixture solution are prepared. Table 2 shows the charge ratio and the weight ratio of chitosan (CS) to polyglutamic acid (γ-PGA), and the particle size and the zeta potential of the biodegradable carrier produced from 5X and 10X mixture solution.

**Table 2**

| Charge ratio CS: γ-PGA | | Weight ratio CS: γ-PGA | Size (nm) | Zeta potential (mV) |
|---|---|---|---|---|
| 5X | 4:1 | 8.52:1.48 | 64.77±0.2546 | 12.5±1.481 |
| | 3:1 | 8.12:1.88 | N/A | N/A |
| | 2:1 | 7.42:2.58 | N/A | N/A |
| 10X | 4:1 | 17.04:2.96 | 84.46±1.662 | 12.8±0.071 |
| | 3:1 | 16.26:3.76 | N/A | N/A |
| | 2:1 | 14.84:5.16 | N/A | N/A |

As shown in Table 1 and Table 2, whatever the biodegradable carrier is produced from mixture solution with one-fold, five-fold, or ten-fold concentration, the biodegradable carrier is stable under the charge ratio of CS to γ-PGA being 4:1. However, the precipitation was happened while the chitosan solution and the polyglumatic acid were mixed under the other charge ratios of CS to γ-PGA, such as 3:1 or 2:1.

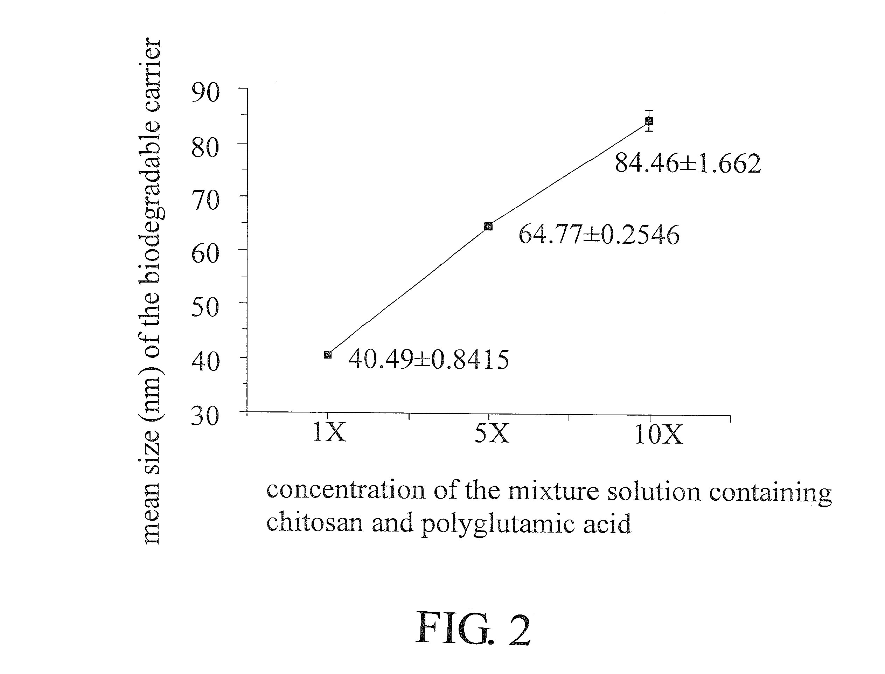

Fig. 2 further shows the analysis results of the mean size of the biodegradable carrier under different concentrations (1X-10X) of the mixture solution containing chitosan and polyglutamic acid in another embodiment of the present invention. As shown in Fig. 2, it is obvious to know that the mean size of the biodegradable carrier is bigger as the concentration of the mixture solution is higher. The concentration of mixture solution shows a linear relationship with the mean size of the biodegradable carrier. Therefore, the biodegradable carrier is obtained through setting the desired zeta potential of the biodegradable carrier first and then adjusting the concentration of the mixture solution for forming the biodegradable carrier with the desired particle size. Table 3 shows the weight ratio of chitosan (CS) to polyglutamic acid (γ-PGA), and the particle size and the zeta potential of the biodegradable carrier produced from mixture solution with 20X - 40X concentration under the charge ratio of CS to γ-PGA being 4:1.

**Table 3**

| Mixture solution concentration | Weight ratio CS: γ-PGA | Size (nm) | Zeta potential (mV) |
|---|---|---|---|
| 1X | 1.704:0.296 | 40.49±0.8415 | 13.2±0.354 |
| 5X | 8.52:1.48 | 64.77±0.2546 | 12.5±1.481 |
| 10X | 17.04:2.96 | 84.46±1.662 | 12.8±0.071 |
| 20X | 34.08:5.92 | 267.0±3.889 | 13.1±0.071 |
| 25X | 42.6:7.4 | 335.2±0.8485 | 13.4±0.071 |
| 30X | 51.12:8.88 | 364.0±4.313 | 12.6±0.424 |
| 35X | 59.64:10.36 | 460.9±5.657 | 12.9±0.424 |

As shown in Table 3, as the mole number of solute in the mixture solution is higher, the mean size of the biodegradable carrier is bigger, but the zeta potential of the biodegradable carrier is similar. It is corresponding to the characteristics of the positive relationship between the particle size of the biodegradable carrier and the mole number of solute in the mixture solution.

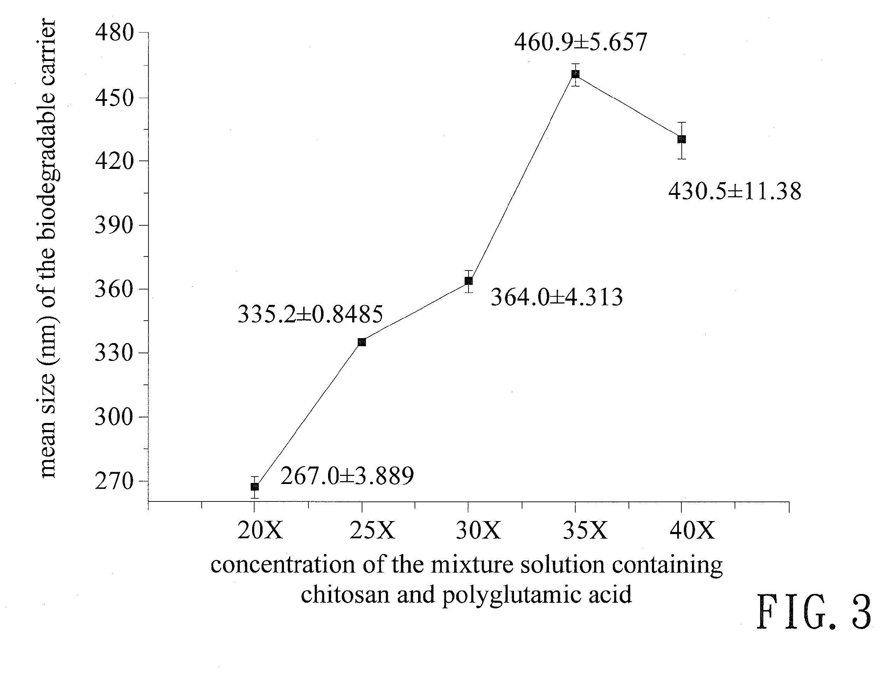

Fig. 3 shows the analysis results of the mean size of the biodegradable carrier under different concentrations (20X-40X) of the mixture solution containing chitosan and polyglutamic acid in the other embodiment of the present invention. The trend of the mean sizes of the biodegradable carrier under different concentrations approximately corresponds to the prospect.

In conclusion, the mix ratio of the first biodegradable macromolecule to the second biodegradable macromolecule in the method for making the biodegradable carrier with adjustable zeta potentials and particle sizes of the present invention could be adjusted according to different applications of the biodegradable carrier. The desired zeta potential of the biodegradable carrier is set first, and then the concentration of the mixture solution is adjusted for making the biodegradable carrier with the desired particle size. In the other word, the zeta potential and the particle size of the biodegradable carrier is adjustable artificially in order that there are different application strategies for meeting the need of the different carried substances with different physical and chemical properties. For example, in the application of targeted drug, the DNA vaccine development and the anti-cancer drug delivery, the carried substance is delivered to the target successfully because the physical and chemical properties of the biodegradable carriers are adjusted. The effect of the drug delivery is greatly increased, and the dose and the side effect of the carried substance are decreased. Moreover, because the particle size of the biodegradable carrier is uniform and the biodegradable carrier is charged, the problem of the aggregation and fusion of the traditional liposome is resolved. In addition, the process for making the biodegradable carrier of the present invention is quite simple and without solvent evaporation step and hydration step so that the cost is greatly decreased. The biodegradable carrier also has better dispersivity in the mixture solution and is not aggregated during the dry process. Therefore, the biodegradable carrier can be kept in the form of a dry powder for resolving the problem that the liposome needs to be kept in the suspension form and further for decreasing the shipping cost.

A pharmaceutical composition is also provided. The pharmaceutical composition comprises a foregoing biodegradable carrier with adjustable zeta potentials and particle sizes, a carried substance, and at least one pharmaceutically acceptable vehicle. The carried substance is held in the biodegradable carrier and is selected from the group consisting of nucleic acid, short peptide, protein drugs, small molecular compounds, viruses, bacterium, and cells. The pharmaceutically acceptable vehicle is a diluting agent, an excipient, an accepting agent, or an analogue. It is noted that the pharmaceutical composition of the present invention can be administered to animals in any existing ways, including oral, nasal, mucosal, topical, dermal, and parenteral administration, wherein the parenteral administration can be intravenous, intraperitoneal, intradermal, subcutaneous, or intramuscular administration. The pharmaceutical composition of the present invention also can be administered via the combination of the foregoing administrations.

The foregoing pharmaceutical composition can be used for producing a vaccine or a drug for treating or preventing hemorrhagic dengue fever or dengue shock syndrome. According to one embodiment, the vaccine or drug for treating or preventing hemorrhagic dengue fever or dengue shock syndrome is a biodegradable high-efficiency dengue vaccine comprising a foregoing biodegradable carrier with adjustable zeta potentials and particle sizes, and a dengue viral protein held in the biodegradable carrier. The dengue viral protein is a dengue virus envelope protein or a dengue virus nonstructural protein. The dengue viral protein has been disclosed in Taiwan Patent application with the Publication No. 201210614 "Dengue vaccine, medicinal composition comprising the same, and nucleotide sequence", the contents of which are hereby incorporated by reference herein. According to an embodiment, the dengue viral protein comprises a nonstructural chimeric protein DJ NS1. The nonstructural chimeric protein DJ NS1 comprises N-terminal amino acid 1-270 of a dengue virus nonstructural protein (DJ NS1) and C-terminal amino acid 271-352 of a Japanese encephalitis virus nonstructural protein (JEV NS1). Moreover, the nonstructural chimeric protein DJ NS1 has more than 90%, even more than 95%, sequence similarity to the SEQ. ID. NO.1. The method for making the foregoing biodegradable high-efficiency dengue vaccine and the effect of the same can be referred to the other US patent application "Biodegradable high-efficiency dengue vaccine, making method of thereof, and pharmaceutical composition comprising the same" filed by the same inventor and on the same date as the present application, the contents of which are hereby incorporated by reference herein, and it is not described here again.

According to the above description, in comparison with the traditional technique, the biodegradable carrier, the making method thereof and the pharmaceutical composition of the present invention has the advantages as following:
1. According to the different utilization, the different mixing ratio of the first biodegradable macromolecule to the second biodegradable macromolecule can be adjustable for controlling the zeta potential and the particle size of the biodegradable carrier. In the other word, according to the different carried substances with different physical and chemical properties, the zeta potential and particle size of the biodegradable carrier is adjustable artificially in order that the need of the different carried substances complies with different application strategies.
2. The zeta potential and the particle size of the biodegradable carrier obtained by the making method of the present invention are more uniform, so that the aggregation and fusion of the traditional liposome due to the large diversity of the size and the zeta potential of the traditional liposome is prevented. Accordingly, the pharmaceutical composition comprising the biodegradable carrier of the present invention produces the effect of the carried substance by penetration or phagocytosis by macrophage easily and releases the carried substance due to its biodegradability.
3. The zeta potential and the particle size of the biodegradable carrier of the present invention is more uniform, so the biodegradable carrier has better dispersivity in the mixture solution and is not aggregated during the dry process. Therefore, the biodegradable carrier can be kept in the form of a dry powder for resolving the problem that the liposome needs to be kept in the suspension form and further for decreasing the shipping cost.
4. The pharmaceutical composition comprises the biodegradable carrier with the zeta potential and size of the biodegradable carrier by artificial, so that not only the release of the carried substance is controlled, but also the pharmaceutical composition can stimulate the specific immune responses (e.g. type 1 and type 2 T cell responses) to enhance the immune responses to the specific antigen for increasing the protective effect of the vaccine.
5. In the method for making a biodegradable carrier of the present invention, the desired zeta potential and particle size of the biodegradable carrier is produced by adjustment of the concentration of the macromolecules, without the solvent evaporation step and the hydration step in the traditional process of the liposome, so that the making cost is greatly decreased.

## Claims

1. A method for making a biodegradable carrier with adjustable zeta potentials and particle sizes comprising the steps of :
preparing a first solution comprising a first biodegradable macromolecule with a first electric property;
preparing a second solution comprising a second biodegradable macromolecule with a second electric property according to a desired zeta potential of a biodegradable carrier and adding the second solution into the first solution to form a mixture solution, wherein the first electric property is opposite to the second electric property for making the biodegradable carrier with the desired zeta potential by attraction force between the different electric properties;
proportionally adjusting the mole number of the first biodegradable macromolecule and the second biodegradable macromolecule in the mixture solution according to a desired particle size of the biodegradable carrier, wherein the particle size of the biodegradable carrier is positively correlated with the mole number of a solute in the mixture solution for making the biodegradable carrier with the desired particle size; and
filtrating the mixture solution after the proportionally adjusting step to obtain the biodegradable carrier.

2. The method as claimed in claim 1, wherein the first macromolecule is heparin or polyglutamic acid, and the first electric property is negatively charged.

3. The method as claimed in claim 1, wherein the second macromolecule is chitosan or collagen, and the second electric property is positively charged.

4. The method as claimed in claim 1, further comprising a step of:
adding a carried substance into the first solution or the second solution for making the carried substance be held in the biodegradable carrier.

5. The method as claimed in claim 4, wherein the carried substance is selected from the group consisting of nucleic acid, short peptide, protein drugs, small molecular compounds, viruses, bacterium, and cells.

6. The method as claimed in claim 5, wherein the electric property of the carried substance is the same as that of solution which the carried substance is added into.

7. A biodegradable carrier with adjustable zeta potentials and particle sizes obtained by the method as claimed in claim 1.

8. The biodegradable carrier as claimed in claim 7, wherein the particle size of the biodegradable carrier ranges from micrometers to nanometers.

9. The biodegradable carrier as claimed in claim 8, wherein the particle size of the biodegradable carrier ranges from 10 µm to 40 nm.

10. The biodegradable carrier as claimed in claim 7, wherein the zeta potential of the biodegradable carrier ranges from +35mv to -35mv.

11. A pharmaceutical composition comprising:
a biodegradable carrier as claimed in claim 7;
a carried substance held in the biodegradable carrier; and
at least one pharmaceutically acceptable vehicle.

12. The pharmaceutical composition as claimed in claim 11, wherein the carried substance is selected from the group consisting of nucleic acid, short peptide, protein drugs, small molecular compounds, viruses, bacterium, and cells.

13. The pharmaceutical composition as claimed in claim 11, which is used for producing a vaccine or a drug for treating or preventing hemorrhagic dengue fever or dengue shock syndrome.
